# EUROPÄISCHE PATENTSCHRIFT

(11) **EP 0 467 010 B1**
(45) Veröffentlichungstag und Bekanntmachung des Hinweises auf die Patenterteilung: **13.12.1995**
(21) Anmeldenummer: 91101485.0
(22) Anmeldetag: 05.02.1991
(51) Int. Cl.: A61M 5/00, B65G 47/90, B67B 1/04, B01L 9/06

(54) **Vorrichtung zur Handhabung von Spritzenzylindern und/oder Karpulen**
Ampulla and/or syringe handling apparatus
Dispositif pour manipuler des seringues et/ou des ampoules

(30) Priorität: 09.07.1990 DE 4021836
(43) Veröffentlichungstag der Anmeldung: 22.01.1992
(73) Patentinhaber: Arzneimittel GmbH Apotheker Vetter & Co. Ravensburg, D-88212 Ravensburg (DE)
(72) Erfinder: Vetter, Helmut, W-7980 Ravensburg (DE); Geprägs, Peter, W-7987 Weingarten (DE)
(74) Vertreter: Dziewior, Joachim, Dipl.-Phys. Dr.

(56) Entgegenhaltungen:
- DE-A- 3 613 489
- DE-C- 166 067
- DE-U- 8 705 814
- DE-U- 8 707 894
- FR-A- 1 183 224
- GB-A- 238 255
- US-A- 2 353 985

## Beschreibung

Die Erfindung betrifft eine Handhabungsvorrichtung mit sich zu ihrem kanülenseitigen Ende hin verjüngenden, ein Mundstück mit einer ringnutförmigen Einschneidung zum Ansatz der Kanüle aufweisenden Spritzenzylindern und/oder Karpulen, mit einer ebenen Trägerplatte sowie einer mit der Trägerplatte über Distanzstücke verbundenen, zur Trägerplatte parallelen Justierplatte, die beide mit zueinander fluchtend angeordneten, paarweise jeweils eine Aufnahme für den Spritzenzylinder bzw. die Karpule bildenden, diese im wesentlichen formschlüssig umgreifenden Aufnahmeaussparungen versehen sind, sowie mit einer Arretierungsplatte, die mit geringem Abstand parallel zur Trägerplatte angeordnet ist, die ferner parallel zu ihrer Oberfläche zwischen einer Verriegelungsstellung und einer Bestückungsstellung verschiebbar geführt und die mit der Trägerplatte auf ihrer der Justierplatte abgewandten Seite verbunden und ebenfalls mit Durchbrüchen versehen ist, die im wesentlichen zur Achse der von den Aufnahmeaussparungen gebildeten Aufnahmen konzentrisch angeordnet sind, wobei die Justierplatte mit zur Trägerplatte abgewandter Richtung vorstehenden Fußteilen versehen ist und bei in die Handhabungsvorrichtung eingesetzten Spritzenzylindern bzw. Karpulen der Rand der Durchbrüche in der Verriegelungsstellung der Arretierungsplatte jeweils dem Spritzenzylinder bzw. der Karpule anliegt, wobei weiter die Handhabungsvorrichtung mit einer Verschlußteilplatte versehen ist, die mit den Aufnahmeaussparungen in der Trägerplatte koaxial zugeordneten Halteaufnahmen für auf das Mundstück aufzusetzende Verschlußteile versehen ist, und wobei die Distanzstücke Führungshülsen für die Verschlußteilplatte bilden.

Eine Vorrichtung dieser Art ist aus der DE-PS 36 13 489 bekannt und hat sich in der Praxis bewährt. Die Arretierungsplatte ist dort an ihren beiden sich gegenüberstehenden Rändern mit einem sich jeweils in Längsrichtung dieses Randes erstreckenden U-Profil versehen, wodurch sie auf die Trägerplatte aufgeschoben werden kann, nachdem die Bestückung mit Spritzenzylindern bzw. Karpulen erfolgt ist. Zur Fixierung der Arretierungsplatte ist am freien Schenkel des U-Profils ein Vorsprung vorgesehen, der eine Anschlagfläche für eines der Distanzstücke bildet. Hierdurch wird eine konzentrische Ausrichtung der Ausnehmungen in der Arretierungsplatte bzw. der Trägerplatte zueinander bewährleistet. Die axiale Ausrichtung der Spritzenzylinder bzw. Karpulen erfolgt hierbei dadurch, daß der Ringflansch an dem dem Mundstück abgewandten Ende der Spritze zwischen der Trägerplatte und der Arretierungsplatte gehalten wird. Die Festlegung der Orientierung der Spritzenzylinder erfolgt daher nicht auf der kritischeren Seite der Mundstücke, so daß Toleranzen in der Länge der Spritzenzylinder zu Schwierigkeiten beim Aufsetzen der Verschlußteile führen kann.

Der Erfindung liegt die Aufgabe zugrunde, eine Vorrichtung der eingangs genannten Art so auszubilden, daß sie bei einer vollautomatische Bestückung und anschließenden Arretierung der darin magazinierten Fertigspritzen bzw. Karpulen deren Festlegung der Orientierung auf verfahrenstechnisch einfache Weise erlaubt.

Diese Aufgabe wird nach der Erfindung dadurch gelöst, daß der Rand der Durchbrüche in der Verriegelungsstellung der Arretierungsplatte am Boden der ringnutförmigen Einschneidung des Mundstücks des Spritzenzylinders bzw. der Karpule anliegt, und daß die Distanzstücke über die Arretierungsplatte hinausstehen.

Der durch die Erfindung erreichte Vorteil besteht im wesentlichen darin, daß bei Arretierung der in der Vorrichtung angeordneten Spritzenzylinder bzw. Karpulen durch einfache Verschiebung der Arretierungsplatte der Ausrichtung in einer Ebene möglich ist. Dies läßt sich auch bei vollautomatischen Arbeitsabläufen auf einfache Weise realisieren.

Die Distanzstücke und die Fußteile sind vorteilhafterweise einstückig miteinander ausgebildet und gemeinsam von zylindrischen Rohrstücken gebildet.

Die Verschlußplatte ist zweckmäßigerweise mit sacklochförmigen Bohrungen zur Aufnahme der Führungshülsen versehen, wodurch sich selbsttätig eine Justierung der Verschlußteilplatte gegenüber den Aufnahmen der Vorrichtung ergibt. In die Führungshülsen können zweckmäßigerweise über ihre Stirnfläche nach oben vorstehende Schraubenfedern eingesetzt sein, die die Verschlußteilplatte in einer solchen Distanz zur Trägerplatte halten, daß die Verschlußteile dem Mundstück der Spritzenzylinder bzw. Karpulen mit Abstand gegenüberstehen. Auf diese Weise können die Spritzenzylinder bzw. Karpulen beispielsweise einer Gefriertrocknung unterworfen und im unmittelbaren Anschluß daran durch Druck auf die Verschlußteilplatte durch die Verschlußteile geschlossen werden.

Die Führungshülsen können zweckmäßigerweise einen radial nach außen vorstehenden Ringflansch aufweisen, der eine Anschlagfläche für die Verschlußplatte bildet. Hierdurch lassen sich Beschädigungen der Verschlußteile vermeiden, die durch zu hohen Druck auf die Verschlußteilplatte auftreten können. Hierbei ist es weiter noch von Vorteil, wenn die der Verschlußteilplatte zugewandte Ringfläche des Ringflansches von einem elastomeren Material gebildet ist.

Weiter ist im Rahmen der Erfindung vorgesehen, daß die Trägerplatte mehrere zur Arretierungsplatte hin vorstehende Führungsbolzen trägt, die jeweils mit einer Ringnut versehen sind, in der die Arretierungsplatte mit einem Langloch geführt ist. Diese Führungsbolzen gewährleisten damit nicht nur die Verschiebung der Arretierungsplatte zwischen der Verriegelungsstellung und der Bestückungsstellung, sondern sorgen darüber hinaus dafür, daß die Arretierungsplatte in einer parallelen Ebene zur Trägerplatte gehalten wird. Der Führungsbolzen kann dabei vorteilhafterweise einen sich zur Justierplatte erstreckenden und dort stirnseitig angeschlossenen Ansatz aufweisen, wodurch auch ein einheitlicher Abstand zwischen der Trägerplatte und der Justierplatte sicher gestellt ist.

Schließlich können die Durchbrüche in der Arretierungsplatte vorteilhafterweise eine langlochförmige Gestalt aufweisen und von zwei sich gegenseitig überlappenden, kreisrunden Bohrungen gebildet sein, wobei der Durchmesser der einen Bohrung dem Querschnitt des Spritzenzylinders bzw. der Karpule und der Durchmesser der anderen Bohrung dem Querschnitt der ringnutförmigen Einschneidung am Mundstück angepaßt ist und wobei die Mittelpunkte beider Bohrungen auf einer zur Verschieberichtung der Arretierungsplatte parallelen Geraden liegt.

Im folgenden wird die Erfindung an einem in der Zeichnung dargestellten Ausführungsbeispiel näher erläutert; es zeigen:
- Fig. 1: einen Schnitt durch den Gegenstand nach Fig. 2 längs der Linie A-B,
- Fig. 2: den Gegenstand nach der Erfindung in Draufsicht, wobei die obere Hälfte mit geöffneter, die untere Hälfte mit geschlossener Arretierungsplatte dargestellt sind.

Die in der Zeichnung dargestellte Vorrichtung dient zur Handhabung von sich zu ihrem kanülenseitigen Ende hin verjüngenden, ein Mundstück 1 zum Ansatz von in der Zeichnung nicht wiedergegebenen Kanülen aufweisenden Spritzenzylindern und/oder Karpulen 2.

Die Vorrichtung besteht im einzelnen aus einer ebenen Trägerplatte 3 sowie einer mit der Trägerplatte 3 über Distanzstücke 4 verbundenen Justierplatte 5. Die Trägerplatte 3 sowie die Justierplatte 5 sind beide mit zueinander fluchtend angeordneten, paarweise jeweils eine Aufnahme für den Spritzenzylinder bzw. die Karpule 2 bildenden Aufnahmeaussparungen 6 versehen, die den Spritzenzylinder bzw. die Karpule 2 im wesentlichen formschlüssig umgreifen.

Weiter ist die Vorrichtung mit einer Arretierungsplatte 7 versehen, die mit der Trägerplatte 3 auf ihrer der Justierplatte 5 abgewandten Seite verbunden und ebenfalls mit Durchbrüchen 8 versehen ist. Diese Durchbrüche 8 sind im wesentlichen zur Achse der von den Aufnahmeaussparungen 6 gebildeten Aufnahmen konzentrisch angeordnet. Im übrigen ist die Justierplatte 5 mit Fußteilen 9 versehen, die in zur Trägerplatte 3 abgewandter Richtung hin vorstehen.

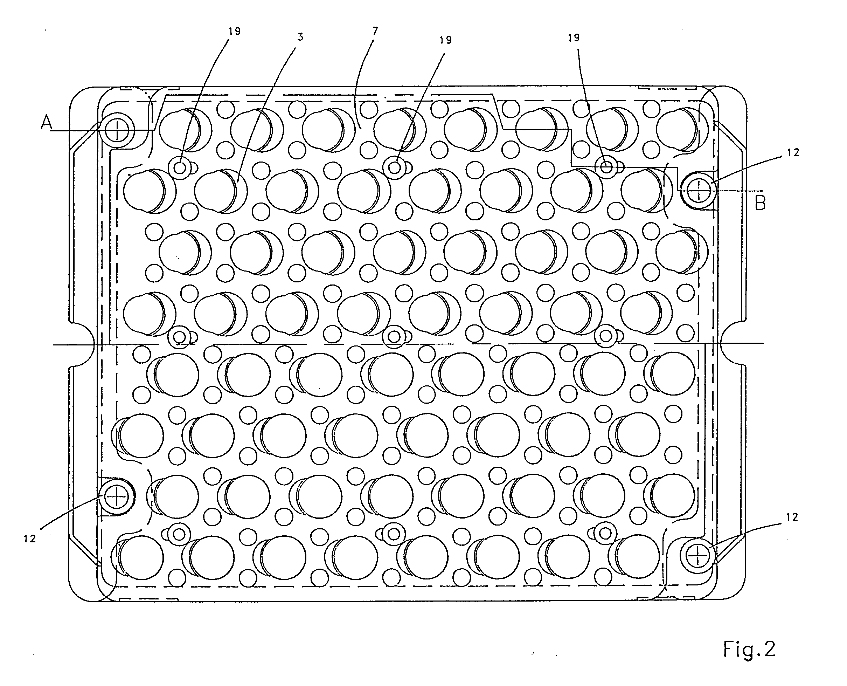

Die Arretierungsplatte 7 ist parallel zu ihrer Oberfläche zwischen einer Verriegelungsstellung und einer Bestückungsstellung verschiebbar geführt, wobei die Verriegelungsstellung in der oberen Hälfte, die Bestückungsstellung in der unteren Hälfte der Fig. 2 dargestellt sind.

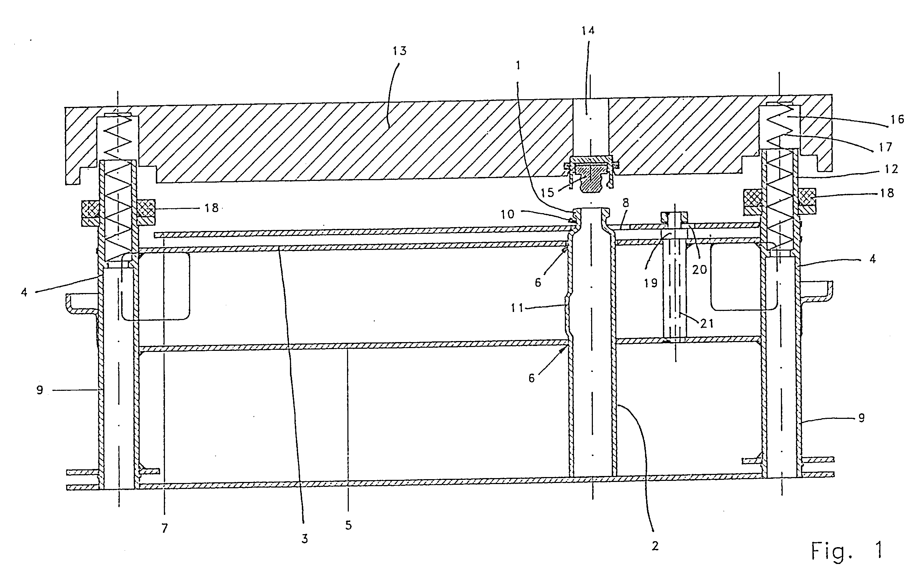

Die Arretierungsplate 7 ist mit solchem Abstand zur Trägerplatte 3 angeordnet, daß bei betriebsmäßig vorgesehener axialer Anordnung der Spritzenzylinder bzw. der Karpulen 2, wie sie aus der Fig. 1 hervorgeht, der Rand der Durchbrüche 8 in der Verriegelungsstellung der Arretierungsplatte 7 jeweils im Bereich der Verjüngung am Mundstück 1 des Spritzenzylinders bzw. der Karpule 2 anliegt.

Der Spritzenzylinder bzw. die Karpule 2 besitzt eine ringnutförmige Einschneidung 10 im Bereich des Mundstücks 1, wie dies bei der Karpule nach Fig. 1 dargestellt ist. Die Arretierungsplatte 7 ist in solchem Abstand zur Trägerplatte 3 angeordnet, daß der Rand der Durchbrüche 8 bei betriebsmäßig vorgesehener axialer Anordnung der Spritzenzylinder bzw. der Karpulen 2 dem Boden der Einschneidung 10 anliegt. Hierdurch wird ohne weitere Maßnahmen direkt die axiale Lage der Karpule 2 bestimmt.

Die Distanzstücke 4 und die Fußteile 9 sind zweckmäßigerweise einstückig miteinander ausgebildet und gemeinsam von zylindrischen Rohrstücken gebildet. Dabei stehen die Distanzstücke 4 auch über die Arretierungsplatte 7 hinaus und bilden Führungshülsen 12 für eine Verschlußteilplatte 13, die mit Halteaufnahmen 14 für Verschlußteile 15 versehen ist. Die Halteaufnahmen 14 in der Verschlußteilplatte 13 sind koaxial zu den Aufnahmeaussparungen 6 in der Trägerplatte 3 angeordnet, so daß sämtliche in der Vorrichtung angeordneten Spritzenzylinder bzw. Karpulen 2 durch einen einzigen Hub der Verschlußteilplatte 13 gegen die Trägerplatte 3 verschlossen werden können. Hierzu ist die Verschlußteilplatte 13 mit sacklochförmigen Bohrungen 16 zur Aufnahme der Führungshülsen 14 versehen, in die im übrigen Schraubenfedern 17 eingesetzt sind, die über die obere Stirnfläche der Führungshülsen 12 hervorstehen. Auf diese Weise werden die Verschlußteile 15 mit Abstand vom Mundstück 1 gehalten, so daß zunächst die bei noch unverschlossenem Spritzenzylinder bzw. Karpule 2 vorzunehmenden Verfahrensschritte durchgeführt werden können.

Die Führungshülsen 12 weisen einen radial nach außen vorstehenden Ringflansch 18 auf, der eine Anschlagfläche für die Verschlußteilplatte 13 bildet. Hierdurch kann der Bewegungshub der Verschlußteilplatte 13 begrenzt werden, so daß Beschädigungen der Verschlußteile 15 bzw. der Spritzenzylinder 2 vermieden werden. Dabei kann, wie dies aus der Fig. 1 hervorgeht, die der Verschlußteilplatte 13 zugewandte Ringfläche des Ringflansches 18 von einem elastomeren Material gebildet sein.

Die Trägerplatte 3 ist mit mehreren zur Arretierungsplatte 7 hin vorstehenden Führungsbolzen 19 versehen, die jeweils eine Ringnut aufweisen, in der die Arretierungsplatte 7 mit einem Langloch 20 geführt ist. Diese Führungsbolzen 19 weisen einen sich zur Justierplatte 5 erstreckenden und dort stirnseitig angeschlossenen Ansatz 21 auf, wodurch eine erhöhte Festigkeit der Vorrichtung erreicht und darüber hinaus eine parallele Ausrichtung zwischen der Trägerplatte 3, der Justierplatte 5 und der Arretierungsplatte 7 erreicht wird.

Die Durchbrüche 8 in der Arretierungsplatte 7 weisen, wie die Fig. 2 zeigt, eine langlochförmige Gestalt auf und sind von zwei sich gegenseitig überlappenden, kreisrunden Bohrungen gebildet. Der Durchmesser der einen, größeren Bohrung ist dabei dem Querschnitt des Spritzenzylinders bzw. der Karpule 2 angepaßt, während der Durchmesser der anderen Bohrung dem Querschnitt der ringnutförmigen Einschneidung 10 am Mundstück 1 entspricht. In der in Fig. 2 unten dargestellten Bestückungsstellung gibt dadurch die Arretierungsplatte 7 den Aufnahmequerschnitt völlig frei. In der in Fig. 2 oben dargestellten Verriegelungsstellung liegt dagegen der Rand der Durchbrüche 8 dem Spritzenzylinder bzw. der Karpule 2 im sich verjüngenden Bereich des Mundstücks 1 an, wobei der diesem Bereich angepaßte Durchmesser der Randlinie des Durchbruchs 8 eine nur punktuelle Berührung mit dem Spritzenzylinder bzw. der Karpule 2 vermeidet. Die Mittelpunkte beider Bohrungen liegen dabei auf einer zur Verschieberichtung der Arretierungsplatte 7 parallelen Geraden.

Im übrigen können in der Zeichnung nicht dargestellte Feststellmittel vorgesehen sein, durch die die Arretierungsplatte 7 in der Verriegelungsstellung und/oder in der Bestückungsstellung festgestellt werden kann. Im einfachsten Fall kann dies durch einen Stift erfolgt, der durch entsprechende Bohrungen der Arretierungsplatte 7 und der Trägerplatte 3 greift. Ebenso können die Feststellmittel aber auch zur einfachen maschinellen Betätigung eingerichtet sein.

## Patentansprüche

1. Handhabungsvorrichtung mit sich zu ihrem kanülenseitigen Ende hin verjüngenden, ein Mundstück (1) mit einer ringnutförmigen Einschneidung zum Ansatz der Kanüle aufweisenden Spritzenzylindern und/oder Karpulen (2), mit einer ebenen Trägerplatte (3) sowie einer mit der Trägerplatte (3) über Distanzstücke (4) verbundenen, zur Trägerplatte parallelen Justierplatte (5), die beide mit zueinander fluchtend angeordneten, paarweise jeweils eine Aufnahme für den Spritzenzvlinder bzw. die Karpule (2) bildenden, diese im wesentlichen formschlüssig umgreifenden Aufnahmeaussparungen (6) versehen sind, sowie mit einer Arretierungsplatte (7), die mit geringem Abstand parallel zur Trägerplatte (3) angeordnet ist, die ferner parallel zu ihrer Oberfläche zwischen einer Verriegelungsstellung und einer Bestückungsstellung verschiebbar geführt und die mit der Trägerplatte (3) auf ihrer der Justierplatte (5) abgewandten Seite verbunden und ebenfalls mit Durchbrüchen (8) versehen ist, die im wesentlichen zur Achse der von den Aufnahmeaussparungen (6) gebildeten Aufnahmen konzentrisch angeordnet sind, wobei die Justierplatte (5) mit zur Trägerplatte (3) abgewandter Richtung vorstehenden Fußteilen (9) versehen ist und bei in die Handhabungsvorrichtung eingesetzten Spritzenzylindern bzw. Karpulen (2) der Rand der Durchbrüche (8) in der Verriegelungsstellung der Arretierungsplatte (7) jeweils dem Spritzenzylinder bzw. der Karpule (2) anliegt, wobei weiter die Handhabungsvorrichtung mit einer Verschlußteilplatte (13) versehen ist, die mit den Aufnahmeaussparungen (6) in der Trägerplatte (3) koaxial zugeordneten Halteaufnahmen (14) für auf das Mundstück (1) aufzusetzende Verschlußteile (15) versehen ist, und wobei die Distanzstücke (4) Führungshülsen (12) für die Verschlußteilplatte (13) bilden, dadurch gekennzeichnet, daß der Rand der Durchbrüche (8) in der Verriegelungsstellung der Arretierungsplatte (7) am Boden der ringnutförmigen Einschneidung des Mundstücks (1) des Spritzenzylinders bzw. der Karpule (2) anliegt, und daß die Distanzstücke (4) über die Arretierungsplatte (7) hinausstehen.

2. Vorrichtung nach Anspruch 1, dadurch gekennzeichnet, daß die Distanzstücke (4) und die Fußteile (9) einstückig miteinander ausgebildet und gemeinsam von zylindrischen Rohrstücken gebildet sind.

3. Vorrichtung nach Anspruch 1 oder 2, dadurch gekennzeichnet, daß die Verschlußteilplatte (13) mit sacklochförmigen Bohrungen (16) zur Aufnahme der Führungshülsen (12) versehen ist.

4. Vorrichtung nach einem der Ansprüche 1 bis 3, dadurch gekennzeichnet, daß in die Führungshülsen (12) über ihre Stirnfläche nach oben vorstehende Schraubenfedern (17) eingesetzt sind.

5. Vorrichtung nach einem der Ansprüche 1 bis 4, dadurch gekennzeichnet,daß die Führungshülsen (12) einen radial nach außen vorstehenden Ringflansch (18) aufweisen, der eine Anschlagfläche für die Verschlußteilplatte (13) bildet.

6. Vorrichtung nach Anspruch 5, dadurch gekennzeichnet, daß die der Verschlußteilplatte zugewandte Ringfläche (18) des Ringflansches von einem elastomeren Material gebildet ist.

7. Vorrichtung nach einem der Ansprüche 1 bis 6, dadurch gekennzeichnet, daß die Trägerplatte (3) mehrere zur Arretierungsplatte (7) hin vorstehende Führungsbolzen (19) trägt, die jeweils mit einer Ringnut versehen sind, in der die Arretierungsplatte (7) mit einem Langloch (20) geführt ist.

8. Vorrichtung nach Anspruch 7, dadurch gekennzeichnet, daß der Führungsbolzen (19) einen sich zur Justierplatte (5) erstreckenden und dort stirnseitig angeschlossenen Ansatz (21) aufweist.

9. Vorrichtung nach einem der Ansprüche 1 bis 8, dadurch gekennzeichnet, daß die Durchbrüche (8) in der Arretierungsplatte (7) eine langlochförmige Gestalt aufweisen und von zwei sich gegenseitig überlappenden, kreisrunden Bohrungen gebildet sind, wobei der Durchmesser der einen Bohrung dem Querschnitt des Spritzenzylinders bzw. der Karpule (2) und der Durchmesser der anderen Bohrung dem Querschnitt der ringnutförmigen Einschneidung (10) am Mundstück (1) angepaßt ist und wobei die Mittelpunkte beider Bohrungen auf einer zur Verschieberichtung der Arretierungsplatte (7) parallelen Geraden liegt.

## Claims

1. Handling device comprising injection cylinders and/or sleeves (2), which taper towards their end associated with the cannula and have a mouth (1) with a notch in the form of an annular groove for attaching the cannula, comprising a plane support plate (3) and an adjusting plate (5) parallel to the support plate and connected to the support plate (3) via spacers (4) - the two plates being provided with holding recesses (6), which are disposed flush with one another and form in pairs a seat for the injection cylinder or sleeve (2) and which encompass the sleeve in a substantially positively locking manner - and comprising a locking plate (7), which is disposed with slight clearance parallel to the support plate (3), which is furthermore guided displaceably parallel to its surface between a locked position and an insertion position and which is connected to the support plate (3) on its side remote from the adjusting plate (5) and is likewise provided with holes (8), which are disposed substantially concentric to the axis of the seats formed by the holding recesses (6), wherein the adjusting plate (5) is provided with tail members (9) projecting in the direction away from the support plate (3) and, in the injection cylinders or sleeves (2) used in the handling device, the edge of the holes (8) in the locked position of the locking plate (7) abuts the injection cylinder or sleeve (2), the handling device further being provided with a sealing member plate (13) provided with seats (14) for sealing members (15) to be mounted on the mouth (1), said seats being associated coaxially with the holding recesses (13) in the support plate (3), and wherein the spacers (4) form guide sleeves (12) for the sealing member plate (13), characterised in that the edge of the holes (8) abuts the base of the annular-groove-shaped notch at the mouth (1) of the injection cylinder or sleeve (2) in the locked position of the locking plate (7), and in that the spacers (4) project beyond the locking plate (7).

2. Device according to claim 1, characterised in that the spacers (4) and the tail members (9) are formed integrally with one another and are formed together from cylindrical tube members.

3. Device according to claim 1 or 2, characterised in that the sealing member plate (13) is provided with blind bores (16) for receiving the guide sleeves (12).

4. Device according to one of claims 1 to 3, characterised in that helical springs (17) are inserted into the guide sleeves (12) so as to project above the end face thereof.

5. Device according to one of claims 1 to 4, characterised in that the guide sleeves (12) have a radially outward-projecting annular flange (18), which forms a stop face for the sealing member plate (13).

6. Device according to claim 5, characterised in that the annular face (18) of the annular flange facing the sealing member plate is formed from an elastomeric material.

7. Device according to one of claims 1 to 6, characterised in that the support plate (3) has a plurality of guide bolts (19) projecting towards the locking plate (7), each being provided with an annular groove, in which the locking plate (7) is guided with a slot (20).

8. Device according to claim 7, characterised in that the guide bolt (19) has a lug (21) extending to the adjusting plate (5) and abutting the end face of said plate.

9. Device according to one of claims 1 to 8, characterised in that the holes (8) in the locking plate (7) have a slot-like shape and are formed by two mutually overlapping, circular bores, the diameter of one bore corresponding to the cross-section of the injection cylinder or sleeve (2) and the diameter of the other bore corresponding to the cross-section of the annular-groove-shaped notch (10) at the mouth (1), and wherein the centre points of the two bores lie on a straight line parallel to the displacement direction of the locking plate (7).

## Revendications

1. Dispositif de manipulation comportant des cylindres de seringues et/ou des ampoules (2) avec un goulot (1) se rétrécissant vers leur extrémité du coté de la canule, pourvu d'une entaille en forme de rainure pour l'adaptation des cylindres de seringues et/ou des ampoules, comportant une plaque de support plane (3) ainsi qu'une plaque d'alignement (5) reliée à la plaque de support (3) par l'intermédiaire de butées d'espacement (4), la plaque de support (3) et la plaque d'alignement (5) étant toutes deux pourvues d'orifices de réception (6) disposés de façon convergente, dont chaque paire forme une ouverture de réception du cylindre de seringue ou de l'ampoule respectivement, et entoure ceux-ci pratiquement de façon crabotée, le dispositif de manipulation comportant en outre une plaque d'arrêt (7) qui est disposée à faible distance de la plaque de support (3), parallèlement à elle, et qui est en outre guidée de façon coulissante, parallèlement à sa surface supérieure entre une position de verrouillage et une position de remplissage, et qui est reliée à la plaque de support (3) sur son côté opposé a la plaque d'ajustement (5) et est pourvue également d'ouvertures (8) qui sont aménagées pratiquement de façon concentrique avec l'axe des ouvertures formées par les orifices de réception (6), la plaque d'alignement (5) étant pourvue d'éléments de base (9) dirigés vers la plaque de support (3) et étant en contact, lorsque les cylindres de seringue ou les ampoules sont respectivement disposées dans le dispositif de manipulation, avec le bord des ouvertures (8) en position de verrouillage de la plaque d'arrêt (7) du cylindre de seringue ou de l'ampoule (2) respectivement, le dispositif de manipulation étant en outre pourvu d'une plaque de fermeture (13), qui est pourvue d'ouvertures de maintien (14) aménagées dans la plaque de support (3) de façon coaxiale avec les orifices de réception (6), pour recevoir des éléments de fermeture (15) destinés à être adaptés sur le goulot (1), et les butées d'espacement (4) formant des douilles de guidage (12) de la plaque de fermeture (13), caractérisé en ce que le bord des ouvertures (8) repose, en position de verrouillage de la plaque d'arrêt (7), contre le fond de l'entaille en forme de rainure du goulot (1) du cylindre de seringue ou de l'ampoule respectivement, et en ce que les butées d'espacement (4) dépassent par rapport à la plaque d'arrêt (7).

2. Dispositif selon la revendication 1, caractérisé en ce que les butées d'espacement (4) et les éléments de base (9) sont formés ensemble d'un seul tenant et obtenus tous deux à partir d'éléments de tubes cylindriques.

3. Dispositif selon la revendication 1 ou la revendication 2, caractérisé en ce que la plaque de fermeture (13) est pourvue d'alésages borgnes (16) pour la réception des douilles de guidage (12).

4. Dispositif selon l'une des revendications 1 à 3, caractérisé en ce que des ressorts hélicoïdaux (17) sont disposés dans les douilles de guidage (12), de façon à dépasser vers le haut par rapport à leur surface frontale.

5. Dispositif selon l'une des revendications 1 à 4, caractérisé en ce que les douilles de guidage (12) comportent une collerette annulaire (18) dépassant radialement vers l'extérieur, formant une surface d'arrêt pour la plaque de fermeture(13).

6. Dispositif selon la revendication 5, caractérisé en ce que la surface annulaire (18) de la collerette orientée vers la plaque de fermeture est constituée d'un matériau élastomère.

7. Dispositif selon l'une des revendications 1 à 6, caractérisé en ce que la plaque support (3) porte plusieurs goujons de guidage (19) faisant saillie vers la plaque d'arrêt (7), chaque goujon de guidage étant pourvu d'une rainure dans laquelle la plaque d'arrêt (7) est guidée par un trou oblong (20).

8. Dispositif selon la revendication 7, caractérisé en ce que le goujon de guidage (19) comporte un épaulement (21) s'étendant vers la plaque d'alignement (5) avec laquelle il est en contact par sa face frontale.

9. Dispositif selon l'une des revendications 1 à 8, caractérisé en ce que les ouvertures (8) de la plaque d'arrêt (7) ont une forme de trou oblong et sont formées de deux alésages circulaires qui se recouvrent mutuellement, le diamètre de l'un des alésages étant adapté à la section transversale du cylindre de seringue respectivement de l'ampoule (2), et le diamètre de l'autre alésage étant adapté à la section transversale de l'entaille en forme de rainure (10) du goulot (1), et les centres des deux alésages étant alignés sur une ligne parallèle à la direction de coulissement de la plaque d'arrêt (7).
